# EUROPEAN PATENT APPLICATION

(11) **EP 3 181 116 A1**
(43) Date of publication of application: **21.06.2017**
(21) Application number: 16002687.8
(22) Date of filing: 19.12.2016
(51) Int. Cl.: A61K 8/37, A61K 8/49, A61Q 1/14, A61Q 17/04, A61Q 19/00

(54) **ESTERS OF ACETALS, COMPOSITION THEREOF, METHODS OF PREPARATION AND MAIN USES THEREOF IN COSMETIC AREA**

(30) Priority: 18.12.2015 BR 102015031992
(71) Applicant: Glycerosolution Quimica, Ltda, CEP 04571-000 Sao Paulo (SP) (BR)
(72) Inventor: Sato, Setsuo, 05706-290 Paraíso do Morumbi, São Paulo - SP (BR); Borges Sato, Marcelo Eiji, 05706-290 Paraíso do Morumbi, São Paulo - SP (BR)
(74) Representative: Juncosa Miro, Jaime

(57) **Abstract**

Esters of acetals, composition thereof, methods of preparation and main uses thereof in cosmetic area refers to a new group of acetal monoesters having excellent solubility power in sunscreens, excellent properties of emollience, spreadability and ease of preparing stable aqueous formulas, completely non-toxic, has no characteristics that generate ocular or cutaneous irritation, which enables its use as solubilizers and emollients in cosmetic formulations for body use, facial sunscreens, makeup removers and component for use in roll-on deodorants.

## Description

### TECHNICAL FIELD

This invention relates to a novel group of monoesters of acetals having, in its structure, the esters of acetals function, produced from glycerin, which give these products excellent properties such as solvency for sunscreens, ease of emulsification, emollience, excellent spreadability without oiliness feature, hydrolytic and chemical stability and low toxicity. These properties make these products excellent candidates as components in cosmetic and personal care formulations like sunscreens, body creams, antiperspirants and deodorants.

### TECHNICAL BACKGROUND

The most commonly used products in the applications mentioned above are those that can solubilize solar ultraviolet radiation protection filters such as dibenzoylmethane derivatives mainly the 4-(I,I-dimethylethyl)-4-metoxydibenzoylmetane (PARSOL 1789) and derivatives of cinnamic acid, in particular 2-ethylhexyl paramethoxycinnamate and octylcinnamate, for the preparation of liquids of stable composition, good spreadability, non-irritability, wash-resistant and especially that generate a comfort sensation in the human skin.

Esters of benzoic acid made with C1 up to C18 carbon chain alcohols, especially those of short chain such as methyl benzoate are quite common; dicapryl ethers, esters of fatty acids such as isopropyl myristate, isopropyl palmitate, octyl octoate. Esters produced from linear or branched carboxylic acids of carbonic chain containing 8 to 18 carbons and acetals produced from glycerin reacted with ketones or aldehydes are the target end products of this invention. Due to the large variety of existing products, the products are selected, in most cases, by the performance, cost and availability of the product in each region. Products from renewable sources may, in some applications, have a higher added value than those from petrochemical source.

Formulations are made using more than one product to achieve the desired effects for each specific need, for example the use of natural antioxidants in cosmetic formulations, which use fatty esters as oil donating agents for the skin. In the industrial area, products and formulations containing effects of lubricity, spreadability, anticorrosive protection, plasticity, adhesiveness, solvency, hydrophilicity, hydrophobicity, are much required by the market.

The cosmetic industry in the production of creams, shampoos and sunscreens uses fatty acid esters such as octyl stearate, isopropyl palmitate, cetyl palmitate, combined with tocopherols to achieve oiliness and protection of the skin and hair.

The domestic and industrial hygiene industry often uses glycols such as butyl glycol or its acetate, monoethylene glycol, monoethylene glycol ether, in cleaning formulations such as detergents, soaps and multi-use cleaners.

In the agricultural industry, it can be verified the use of ethylhexyl lactate, lactamides, toluol, xylol, methyl caprylate, methyl oleate, methyl linoleate, isoparaffins, triacetin, isophorones, citric acid esters, lactic acid esters, Maleic acid, propylene glycol and its diesters, dimethicone, phthalic acid diesters, etc.

Sunscreens and a variety of additives used in cosmetic formulations are solid substances to solubilize. Atoxic products that solubilize and provide good spreadability are the most desired for this type of market. Usually, there are several components looking for the most balanced formula considering the regions, their different ethnicities, climates and economic power so that a greater number of users can benefit from a safe and accessible formula.

Patent US 5,783,173 describes sunscreens and their stable formulations, which use the derivatives of benzoic acid as solubilizer and emollient.

Patent US 20200291010 describes the development of new esters of benzoic acid using branched alcohols whose purpose is to prepare formulations of sunscreens, deodorants and antiperspirants.

Patent US 7,166,739 also describes new esters for cosmetic purposes.

### OBJECTIVES OF THE INVENTION

The objective of this invention is to create with the glycerin derivatives, motivated by the growing world production thereof, new solubilizing agents of sunscreen since they are the components of greater difficulty of solubilization.

### DETAILED DESCRIPTION OF THE OBJECT

This invention refers to U.S. Patent No. 20140350269 to the same author, describing the synthesis and applications of the esters of acetals, which belongs to the development family previously described in patents PI 0603912-0 and PI 0703673-6 by the same author, where the acetals can be formed in situ, i.e., acetals and esters are formed at the same time and whose glycerin used is the purified glycerin obtained according to the patent BR 1020120015846, although distilled glycerin or 'blond' glycerin treated as a market standard can be used in the syntheses of the product contemplated in this invention.

This invention is directed to the use of a specific group of esters of acetals for use in formulations for personal use. It has been shown that esters of acetals containing 12 to 24 total carbons originating from the carbonic chain of the carboxylic acid added to the carbons from the acetals have excellent solubilizing power, are non-irritating to human or ocular skin, are nontoxic, have excellent chemical, hydrolytic stability, good lubricity, low oiliness and generate stable aqueous emulsions and dispersions. These are the initial requirements for candidates for the chemical group called solubilizers and emollients in the cosmetic industry.

These esters are represented by the chemical structure (glycerin acetal esters) below: 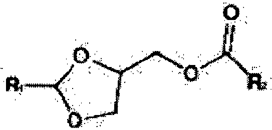 where R1 represents 3 to 8 carbons and R2 represents 8 to 18 carbons.

An object of the invention is the use of esters of acetals as cosmetic agents, wherein the substances are glycerin acetal esters, more particularly monoesters of acetals prepared from oils and/or coconut fatty acids, babassu, palmiste, represented by the chemical structure:

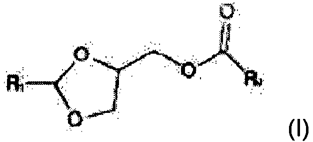

Where:
R1 are the reaction products of glycerin with aldehydes and/or ketones containing from 1 to 8 carbons, more particularly from 3 to 4 carbons; and
R2 are the residue of monocarboxylic acids from 2 to 18 carbon atoms, linear or branched, more particularly the monocarboxylic acids with 12 carbons or more known as lauric acids; such carboxylic acids can be mixtures of high purity obtained by the process of enrichment as distillation, extraction or crystallization.

A further object of the invention is a method of preparation of esters of formula (I), wherein they are obtained through the esterification or transesterification, wherein acetals can be prepared previously or in situ; the reactions of formation of acetals and esters are formed concurrently.

In a preferred method of preparation of glycerin acetals of formula (I), they are prepared from glycerin of several degrees of purity, more particularly purified glycerin with content above 90% of purity.

A further object of the invention is the use of glycerin acetal monoesters of formula (I) in formulations of personal care. Preferred uses are selected from at least one of: use as sunscreen solubilizers against the sun radiation, use as emollients, use as spreadability agents in creams and protective body and face solutions against sun radiation are aqueous and oily. An object of the invention are therefore formulations for personal care, containing compounds of formula (I) as above defined.

Glycerin acetal esters above disclosed may be used as a component in cosmetic formulations for makeup removal. An object of the invention are cosmetic formulations for makeup removal containing glycerin acetals of formula (I).

Glycerin acetal esters of claims 1-3, wherein they are used as components of formulations of antiperspirant deodorants. Formulations of antiperspirant deodorants containing the compounds of formula (I) are another object of the present invention.

Table 1 below shows the basic physical and chemical features of cocoil glyceryl acetal used in the solubilizations and formulations tests of the sunscreens.

**Table 1 (cocoil glyceryl acetal - batch HNC214):**

| Aspect | Transparent liquid |
|---|---|
| Active substance | 99.8% |
| Odor | characteristic |
| Freezing point (Celsius) | -3 |
| Flash point (Celsius) | 155 |
| Density 25 Celsius | 0.925 |
| Viscosity@40Celsius | 8.2 centistokes (cS) |
| Acidity index (mgkoh/g) | 0.2 |
| Saponification index (mgkoh/g) | 178 |
| Gardner color | < 1 |

Table 2 below shows solubility of 3 sunscreens obtained in the Market using cocoil glyceryl acetal as a Solubilizing agent.

**Table 2:**

| Name of the sunscreen filter | Solubility at 25°C grams/100 grams of cocoil glyceryl acetal |
|---|---|
| Ethylhexyl Triazone | 16.0 |
| Diethylamino hydroxybenzoi hexyl benzoate | 18.0 |
| 4-(1,1-dimethylethyl)-4-methoxydibenzoylmethane | 14.0 |

In the tests of oral toxicity, the product was classified as class 5 (non-toxic) according to the rules of GHS - "Globally Harmonized Classification for Chemical Substances and Mixtures".

In the tests of ocular irritation, the product has been classified as non-irritating according to the rules of OECD and GHS.

In the tests of skin irritation, the product has been classified as non-irritating in two tests according to the rules of OECD and GHS, and one of the tests found mild dermal irritation.

Two new batches of cocoil glyceryl acetal were sent to confirm the tests of dermal irritation.

In the following examples, the cocoil glyceryl acetal substance proposed in the invention, was tested in formulations of protective body lotion for ultraviolet radiation (example 1), free-oil face sunscreen (example 2), make up remover (example 3) and formulation of antiperspirant deodorant (example 4), all being compared with products commercially used in the market: C12 - C15 Alkyl Benzoate, Dibutyl adipate, Dicaprylyl carbonate.

**Example 1: Body Lotion EPS 30**

| Phase | INCI Composition - names | Test A | Test B | Test C | Test D |
|---|---|---|---|---|---|
| I | Xanthan Gum | 0.10 | 0.10 | 0.10 | 0.10 |
| I | Glycerin | 1.00 | 1.00 | 1.00 | 1.00 |
| II | Disodium EDTA | 0.05 | 0.05 | 0.05 | 0.05 |
| II | Acrylates / Beheneth-25 Methacrylate Copolymer | 0.50 | 0.50 | 0.50 | 0.50 |
| II | Water | 100 | 100 | 100 | 100 |
| III | Diethylamino Hydroxybenzoyl Hexyl Benzoate | 5.50 | 5.50 | 5.50 | 5.50 |
| III | Titanium Dioxide (and) Hydrate Silica (and) Dimethicon/Methicone Copolymer (and) Aluminum Hydroxide. | 2.00 | 2.00 | 2.00 | 2.00 |
| III | Ethylhexyl Methoxycinnamate | | 9.00 | 9.00 | 9.00 |
| III | Cocoil Glyceryl Acetal | 3.00 | --- | --- | --- |
| III | C12-C15 Alkyl Benzoate | --- | 3.00 | --- | --- |
| III | Dibutyl Adipate | --- | --- | 3.00 | --- |
| III | Dicaprylyl Carbonate | --- | | | 3.00 |
| III | Caprylic Capric Triglyceride | 3.00 | 3.00 | 3.00 | 3.00 |
| III | Cetearyl Alcohol | 2.00 | 2.00 | 2.00 | 2.00 |
| III | Glyceryl Stearate | 2.00 | 2.00 | 2.00 | 2.00 |
| III | Sodium Cetearyl Sulfate | 2.00 | 2.00 | 2.00 | 2.00 |
| III IV | BHT | 0.10 | 0.10 | 0.10 | 0.10 |
| V | Aminomethyl Propanol | qs | qs | qs | qs |
| | Methylene Bis Benzotriazolyl Tetramethylbutylphenol, water, Decylglucoside, Propylene Glycol, xanthan gum. | 0.30 | 0.30 | 0.30 | 0.30 |
| V | Water | 0.30 | 0.30 | 0.30 | 0.30 |
| VI | Phenoxyethanol (and) Methylparaben (and) Ethylparaben (and) Propylparaben (and) Butylparaben (and) Isobutylparaben | 0.50 | 0.50 | 0.50 | 0.50 |
| VI | Fragrance | 0.60 | 0.60 | 0.60 | 0.60 |

### Process:

1. Weigh the components of phase I, add to Phase II and heat at 80 - 85°C.
2. Weigh the components of phase III, homogenize and heat at 80 - 85°C.
3. Pour phase I + II over III under constant stirring.
4. Add Phase IV under stirring up to pH = 6.5 - 7.0, when homogeneous emulsion formation will occur.
5. Separately, premix Phase V and when temperature is below 40°C, add to the emulsion under stirring.
6. Add the components of Phase VI, one by one and homogenize. Adjust the pH (6.5 - 8.5) if necessary.

### Sensory Evaluation - Purpose

- Sensory evaluation is a valuable tool for describing creams, lotions, oils, or other compounds from the sensorial point of view.
- Several parameters are evaluated by a panel trained under conditions of use.
- Along with other physical methods (Corneometer, TEWL) which helps to describe the claims of cosmetic application.
- Procedure:
- Heated room 21 - 24 °C.
- Panel trained with 11 volunteers.
- Evaluation is initially effected é in 1 min and in 3 minutes after the product application.
- It is applied 0.50 g of the test product in the forearm scrubbing with the opposite hand, using the 4 fingers.
- It is evaluated the spreadability, absorption, oiliness, softness and smoothness. We use the scales from 1 to 5; wherein 1 = Bad and 5 = Great.

The formulations B, C and D were subjected to the sensorial panel, compared to formula A, where the results are listed in the Table I below:

| Evaluation | Test A | Test B | Test C | Test D |
|---|---|---|---|---|
| Spreadability | 4 | 2 | 3 | 4 |
| Absorption | 5 | 3 | 3 | 4 |
| Oiliness | 5 | 3 | 4 | 4 |
| Softness | 4 | 4 | 4 | 4 |
| Smoothness | 5 | 3 | 4 | 4 |

The results show that in this formulation the sensorial tests were superior to the market products.

**Example 2: Oil-free face protector.**

| Phase | Composition -INCI names | Test 1 | Test 2 | Test 3 | Test 4 |
|---|---|---|---|---|---|
| I | Diethylamino Hydroxybenzoyl Hexyl Benzoate | 5.00 | 5.00 | 5.00 | 5.00 |
| I | Ethylhexyl Methoxycinnamate | 8.50 | 8.50 | 8.50 | 8.5 |
| I | Cocoil Glyceryl Acetal | 2.00 | --- | --- | --- |
| I | C12-C15 Alkyl Benzoate | --- | 2.00 | --- | --- |
| I | Dibutyl Adipate | --- | --- | 2.00 | --- |
| | Dicaprylyl Carbonate | --- | --- | --- | 2.00 |
| I II | Sodium Acrylates Copolymer and Paraffinum Liquid and PPG-1 Trideceth-6 Disodium EDTA | 3.00 0.05 | 3.00 0,05 | 3.00 0.05 | 3.00 0.05 |
| II | Water | 100 | 100 | 100 | 100 |
| III | Methylene Bis Benzotriazolyl Tetramethylbutylphenol, water, Decylglucoside, Propylene Glycol, xanthan gum | 6.0 | 6.00 | 6.00 | 6.00 |
| III | Water | 6.00 | 6.00 | 6.00 | 6.00 |
| IV | Phenoxyethanol (and) Methylparaben (and) Ethylparaben (and) Propylparaben (and) Butylparaben (and) Isobutylparaben | 0.50 | 0.50 | 0.50 | 0.50 |
| IV | Cyclomethicone | 3.00 | 0.50 | 0.50 | 0.50 |
| IV | Aluminum Starch Octenylsuccinate | 3.00 | 3.00 | 3.00 | 3.00 |
| IV | Fragrance | 0.30 | 0.30 | 0.30 | 0.30 |

### Procedure:

1. Mix Phase I and Phase II and heat at 80 - 85°C.
2. Mix them under constant stirring until emulsion formation.
3. Premix Phase III and when temperature is below 40°C, add to the emulsion under constant stirring.
4. Add the components of Phase IV, one by one and homogenize. Adjust the pH (6.5 - 8.5) if necessary.

The formulations 2, 3 and 4 were subjected to the sensorial panel, compared to the formula 1, where the results are listed in the table below. The protocol used was the same described above, used in the body lotion.

| Evaluation | Test 1 | Test 2 | Test 3 | Test 4 |
|---|---|---|---|---|
| Spreadability | 5 | 3 | 3 | 4 |
| Absorption | 4 | 3 | 4 | 4 |
| Oiliness | 5 | 3 | 4 | 4 |
| Softness | 4 | 4 | 4 | 4 |
| Smoothness | 5 | 3 | 4 | 4 |

**Example 3: Makeup remover**

| Phase I | Composition INCI names Glyceryl Stearate (and) Ceteareth-20(and) Ceteareth12 (and) Cetearyl Alcohol (and) Cetyl Palmitate | Test E 6.00 | Test F 6.00 | Test G 6.00 | Test H 6.00 |
|---|---|---|---|---|---|
| I | Cetearyl Alcohol | 0.50 | 0.50 | 0.50 | 0.50 |
| I | Light liquid paraffin | 6.00 | 6.00 | 6.00 | 6.00 |
| I | Isopropyl Palmitate | 6.0 | 6.00 | 6.00 | 6.00 |
| I | Cocoil Glyceryl Acetal | 3.00 | --- | --- | |
| I | C12-C15 Alkyl Benzoate | --- | 3.00 | --- | |
| I | Dibutyl Adipate | --- | --- | 3.00 | --- |
| I | Dicaprylyl Carbonate | --- | --- | --- | 3.00 |
| II | Preservative | 0.30 | 0.30 | 0.30 | 0.30 |
| II | Water (qs) | 100 | 100 | 100 | 100 |

### Procedure:

1. Mix Phase I and heat at 80 - 85"C.
2. Mix Phase II and heat at 80 - 85°C.
3. Under stirring, add Phase II over Phase I.
4. Cool down under stirring at 30°C and discharge.

The performance test was performed, where the performance of the emollient was evaluated in the effectiveness of the removal power of lipstick and makeup.

The Makeup Remover formulations were evaluated according to the protocol below:
1. Apply the lipstick on the forearm, making a risk in the same.
2. Apply foundation, making a risk in the same.
3. Let it rest for 10 min.
4. Carry out the removal with a non-woven fabric, moistened with 2 g of the makeup remover to be tested.
5. Evaluate the same, mentioning whether the emollient increased or decreased the power of removal. Considering note 1, as being decreased and 2, increased.
6. Evaluate the sensory in the requirements of softness and smoothness. We used scales from 1 to 5; wherein 1 = Bad and 5 = Great.

| Evaluation | Test E | Test F | Test G | Test H |
|---|---|---|---|---|
| Removal Power of Makeup | 2 | 2 | 2 | 2 |
| Softness | 5 | 4 | 4 | 4 |
| Smoothness | 5 | 3 | 4 | 4 |

**Example 4 - Roll On Deodorant**

| Phase | Composition INCI names | Test I | Test J | Test K | Test L |
|---|---|---|---|---|---|
| I. | Glyceryl Stearate (and) Ceteareth-20(and) Ceteareth12 (and) Cetearyl Alcohol (and) Cetyl Palmite | 2.1 | 2.1 | 2.1 | 2.1 |
| | Ceteareth-20 | 1.9 | 1.9 | 1.9 | 1.9 |
| | Glycerin Mono-Distearate | 2.0 | 2.0 | 2.0 | 2.0 |
| | Behenyl Alcohol | 1.0 | 1.0 | 1.0 | 1.0 |
| | Ethylhexyl Stearate | 1.7 | 1.7 | 1.7 | 1.7 |
| | Cocoil Glyceryl Acetal | 1.7 | --- | --- | --- |
| | C12-C15 Alkyl Benzoate | --- | 1.7 | --- | --- |
| | Dibutyl Adipate | --- | --- | 1.7 | --- |
| | Dicaprylyl Carbonate | --- | --- | --- | 1.7 |
| II. | Disodium Edta | 0.05 | 0.05 | 0.05 | 0.05 |
| | Distilled Water (qs) | 100.0 | 100.0 | 100.0 | 100.0 |
| III. | Aluminium Sesquichlorhydrate | 40.0 | 40.0 | 40.0 | 40.0 |
| IV. | Fragrance | 0.1 | 0.1 | 0.1 | 0.1 |

### Process:

1. Weight Phase I, heat it until 80°0.
2. Weight Phase II, heat it until 80°C, add phase V over phase II.
3. Pour phases II + V over phase I.
4. Cool the emulsion until 45°C, add phase III and then phase IV.

### Protocol:

- 10 volunteers.
- Application in the armpit.
- It is evaluated the spreadability, absorption, oiliness, softness and smoothness. We use scales from 1 to 5; wherein 1 = Bad and 5 = Great.

The formulations J, K and L were subjected to the sensorial panel, compared to the formula I, where the results are listed in the table below.

| Evaluation | Test I | Test J | Test K | Test L |
|---|---|---|---|---|
| Spreadability | 5 | 3 | 3 | 4 |
| Absorption | 5 | 3 | 4 | 4 |
| Oiliness | 5 | 3 | 4 | 4 |
| Softness | 5 | 4 | 4 | 4 |
| Smoothness | 5 | 4 | 4 | 4 |

In all these formulations, Cocoil Gyceryl Acetal demonstrated its solubilizing and/or emollient features in these cosmetic applications.

## Claims

1. Esters of acetals and their use as cosmetic agents, wherein the substances are glycerin acetal esters, more particularly monoesters of acetals prepared from oils and/or coconut fatty acids, babassu, palmiste, represented by the chemical structure: 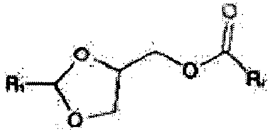 Where:
R1 are the aldehydes and/or ketones containing from 1 to 8 carbons, more particularly from 3 to 4 carbons; and
R2 are monocarboxylic acids from 2 to 18, linear or branched, more particularly the monocarboxylic acids with 12 carbons or more commercially known as lauric acids; such carboxylic acids can be mixtures of high purity obtained by the process of enrichment as distillation, extraction or crystallization.

2. Use of esters of acetals as cosmetic agents, wherein the substances are glycerin acetal esters, more particularly monoesters of acetals prepared from oils and/or coconut fatty acids, babassu, palmiste, represented by the chemical structure: 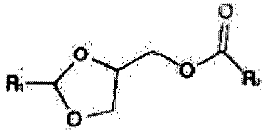 Where:
R1 are the reaction products of glycerin with aldehydes and/or ketones containing from 1 to 8 carbons, more particularly from 3 to 4 carbons; and
R2 are the residue of monocarboxylic acids from 2 to 18 carbon atoms, linear or branched, more particularly the monocarboxylic acids with 12 carbons or more known as lauric acids; such carboxylic acids can be mixtures of high purity obtained by the process of enrichment as distillation, extraction or crystallization.

3. A method of preparation of esters of claim 1, wherein they are performed through the esterification or transesterification, wherein acetals can be prepared previously or *in situ;* the reactions of formation of acetals and esters are formed concurrently.

4. A method of preparation of glycerin acetals of claim 1, wherein they are prepared from glycerin of several degrees of purity, more particularly purified glycerin with content above 90% of purity.

5. Use of glycerin acetal monoesters of claim 1 in formulations of personal care, said use being selected from at least one of: use as sunscreen solubilizers against the sun radiation, use as emollients, use as spreadability agents in creams and protective body and face solutions against sun radiation are aqueous and oily.

6. Glycerin acetal esters of previous claims, wherein they are used in cosmetic formulations for makeup removal.

7. Glycerin acetal esters of claims 1-3, wherein they are used as components of formulations of antiperspirant deodorants.
